# EUROPEAN PATENT SPECIFICATION

(11) **EP 3 789 068 B1**
(45) Date of publication and mention of the grant of the patent: **31.01.2024**
(21) Application number: 19929114.7
(22) Date of filing: 11.06.2019
(51) Int. Cl.: A61M 16/04

(54) **NOVEL LARYNGEAL MASK AIRWAY DEVICE**
NEUARTIGE LARYNXMASKE
NOUVEAU DISPOSITIF RESPIRATOIRE À MASQUE LARYNGÉ

(30) Priority: 15.05.2019 CN 201910403666
(43) Date of publication of application: 10.03.2021
(73) Proprietor: Henan Tuoren Medical Device Co., Ltd, Xinxiang, Henan 453400 (CN); Zhou, Gary Xingguang, Xinxiang, Henan 453400 (CN)
(72) Inventor: SUN, Bao, Xinxiang, Henan 453400 (CN); ZHANG, Jianchao, Xinxiang, Henan 453400 (CN); ZHOU, Yancong, Xinxiang, Henan 453400 (CN); LIANG, Cunxia, Xinxiang, Henan 453400 (CN); YU, Bin, Xinxiang, Henan 453400 (CN); XU, Zhenfeng, Xinxiang, Henan 453400 (CN); LI, Dongtao, Xinxiang, Henan 453400 (CN); ZHOU, Gary Xingguang, Xinxiang, Henan 453400 (CN)
(74) Representative: Nordmeyer, Philipp Werner
(86) International application number: PCT/CN2019/090708
(87) International publication number: WO 2020/228085

(56) References cited:
- EP-A1- 3 549 627
- WO-A1-2014/166136
- CN-A- 106 178 209
- CN-A- 106 178 209
- CN-U- 205 252 245
- CN-U- 205 252 245
- CN-U- 205 814 822
- CN-U- 206 167 560
- CN-U- 207 871 238
- CN-U- 207 871 238
- US-A1- 2016 114 117

## Description

### FIELD

The present application relates to the technical field of medical instruments, and in particular to a laryngeal mask airway.

### BACKGROUND

A laryngeal mask, as an effective means for airway management, can establish a safe airway especially in general anesthesia surgery. It has been widely used in clinical practice. However, a traditional single-tube laryngeal mask is coupled with the respiratory tract in a poor sealing, and it is easily displaced and cannot effectively isolate the respiratory tract from the digestive tract. Moreover, the traditional single-tube laryngeal mask may also cause flatulence, and in severe cases, it may be complicated by reflux or aspiration, which limits its application in clinical anesthesia. Clinical research shows that the laryngeal mask with esophageal drainage has the advantages of simple operation, high success rate of laryngeal mask implantation and prevention of aspiration by mistake. In addition, in the traditional laryngeal mask, the airway tube cavity thereof is naturally oriented along the length direction of the laryngeal mask. Therefore, when the laryngeal mask is sealed and joined to the throat, there is a certain deviation between the direction of the inner vent of the mask body and the alignment of the glottis under special circumstances. When the traditional intubated laryngeal mask is used for intubation through laryngeal mask airway, the front end of endotracheal intubation may still damage glottis and airway wall.

It is found that the airway tube cavity of the traditional laryngeal mask has the same cross-section throughout its length, which is mainly a circular cross-section with the same diameter. The diameter of the circular cross-section is approximately equal to the diameter of the endotracheal intubation, so as to accurately guide and form sealing. However, because the longitudinal length of the airway tube cavity is relatively long and the diameter is approximately the same, it bears relative large frictional force over the entire length. Therefore, it is difficult to insert the endotracheal intubation, and it is easy to cause injury and pain to the patient.

In the traditional laryngeal mask, the contour of the vent at the patient end (the inner side of the mask body) of the laryngeal mask airway tube cavity is all in a plane, and ridge protrusions and auxiliary vent grooves/ports are not designed. When the traditional laryngeal mask is joined to the throat in a sealing manner, due to the retroflex of epiglottis and the filling of peripheral tissues, there is a risk that the vent at the patient end of the airway tube cavity may be blocked, resulting in suffocation of the patient.

In addition, in the clinical use of the laryngeal mask, it is often necessary to drain the patient's esophagus to draw out the reflux fluid from the stomach. The traditional laryngeal mask does not integrate the esophageal drainage tube in an integrated structure, so it cannot successfully establish a safe airway while achieving esophageal drainage.

In addition, even if the esophageal drainage tube is combined with the laryngeal mask, it can only drain the reflux fluid from the stomach and esophagus. However, in the use of laryngeal mask, there may be oral secretion and even bleeding around the airbag cover. Since the esophageal drainage tube is inserted into the esophagus, it cannot drain these oral secretions and bleeding.

CN 206 167 560 U discloses a visual laryngeal mask air vent including a cleaning device to keep a camera lens of a visual device of the mask air vent clean.

### SUMMARY

Therefore, the embodiment of the present application provides a laryngeal mask airway, which integrates the functions of visual monitoring, airway breathing, esophageal drainage, throat drainage and intubation through the laryngeal mask, can improve the visual airway monitoring management, and avoid the defect of damaging the airway during blind intubation.

In order to achieve the above object, the embodiment of the present application provides the following technical solutions.

The embodiment of the present application provides a laryngeal mask airway, including an airbag cover and an extension body. The extension body is shaped according to the natural curvature of the oral cavity and the throat airway and is arc-shaped, and has a proximal end for extending into the oral throat and a distal end located outside the oral cavity; the extension body has an airway tube cavity extending from the distal end to the proximal end, thereby forming a distal vent and a proximal vent respectively. The airbag cover is arranged at the proximal end and has a cover belly forming a concave space and a cover back on the opposite side. The cover belly, having the concave space, of the airbag cover is communicated with the proximal vent of the airway tube cavity. Where the proximal vent is tilted toward the cover belly with respect to the axis at the proximal end of the airway tube cavity, thereby forming a vent tilted portion. The laryngeal mask airway further includes a video tube cavity, and the video tube cavity extends from the distal end to the proximal end in the extension body, thereby forming a distal end opening of video tube cavity at the distal end and a blind end transparent window at the proximal end. The blind end transparent window is located at one side of the proximal vent. The laryngeal mask airway further includes an esophageal drainage tube cavity. The esophageal drainage tube cavity extends from the distal end to the cover back of the airbag cover in the extension body, thereby forming a proximal esophageal drainage port and a distal esophageal drainage port. The proximal part of the esophageal drainage tube cavity is embedded in the inner side of the esophageal drainage port, and the proximal end of the cavity is matched with a flexible material to perform a sealing anastomosis with the esophageal opening of the laryngopharynx. The distal end is opened outside the lip, and the proximal esophageal drainage port of the esophageal drainage tube cavity is formed in an open first groove on the upper side of the cover back; a cover top drainage port is formed at the top end of the cover back of the airbag cover, and the first groove and the cover top drainage port are in fluid communication with each other. The laryngeal mask airway further includes a pharyngeal tube. The pharyngeal tube is made of flexible material. The pharyngeal tube and the airway tube cavity are not communicated with each other, and are independent of the esophageal drainage tube cavity. It includes a main body section integrated in the extension body and a free section extending from the distal end of the extension body to the outside of the extension body. A tail end of the free section is matched with a standard drainage interface, and the main body section extends from the distal end to the proximal end in the extension body, and forms a proximal pharyngeal opening at the proximal end. In the extension body, the main body section is located on the side opposite to the esophageal drainage tube cavity in the left-right direction, and the proximal pharyngeal opening is shaped into an open second groove located on the inner side of the cover edge on the back side of the airbag cover; the first groove, the second groove and the cover top drainage port are in fluid communication with each other to form a three-way structure.

Preferably, the angle included between the vent tilted portion tilts relative to the axis at the proximal end of the airway tube cavity is between 15 and 45 degrees.

Preferably, a distal end of the vent tilted portion adopts a streamlined arc design and an endotracheal intubation guide groove structure is formed at a proximal end of the vent tilted portion, which can assist in guiding the endotracheal intubation into the patient's airway through the airway tube cavity.

Preferably, a part of a side wall surface of the proximal vent is concave in a direction toward the cover back, thereby forming an auxiliary vent groove.

Preferably, the proximal vent and the distal vent of the airway tube cavity are both circular in cross section and have the same diameter. A cross-sectional dimension of an intermediate portion between the distal vent and the proximal vent is preferably larger than a cross-sectional dimension of the proximal vent of the airway tube cavity.

Preferably, the intermediate portion between the proximal vent and the distal vent has an elliptical shape in cross section and a short axis length of the elliptical shape is equal to the diameter of the circle.

Preferably, the airbag cover is an inflatable airbag cover and further includes an inflation tube connected to the airbag cover, the airbag cover is made of flexible material, and the hardness of which is lower than the Shore A hardness of 30.

The embodiments of the present application has the following advantages: according to the laryngeal mask airway provided by the embodiment of the present application, the laryngeal bag is made into a bag which conforms to the anatomical structure in advance, where the airway is simple, effective, safe and convenient in clinical use, and has high cost-effectiveness in production and manufacture. In addition, when the traditional artificial airway can't improve the quality of airway management and hinder the operation, in order to effectively meet the requirements of various clinical situations, such as ophthalmology, otolaryngology, ENT, or head surgery, neck surgery, maxillary surgery, this type of laryngeal mask is preferred. The reason is that it can flexibly and effectively open the surgical field to continue to provide breathing for the patient without interfering with the operation, and can effectively provide multiple airway safety management programs. In summary, laryngeal mask airways have many advantages in certain applications, but we have found that in many applications, the use of inter-buccal fixators can also be a disadvantage. For example, in the use of traditional laryngeal mask, when inserting endotracheal intubation or esophageal drainage tube through laryngeal mask, doctors cannot see the human tissue in front of the insertion path, so it is easy to cause injury to patients. However, with the improvement of medical clinical requirements, the demand for throat real-time monitoring and intubation replacement function is increasingly strong, and the traditional laryngeal mask has no backpack drainage function in otolaryngology clinical surgery, which greatly affects the clinical application effect of laryngeal mask and limits the cross application among departments. The development of visual and multipurpose drainage laryngeal mask greatly improves the effectiveness and safety of clinical application, and is easy to popularize and apply in clinical anesthesia.

The present invention is disclosed by appended claim 1. Further aspects are object of the dependent claims.

### BRIEF DESCRIPTION OF THE DRAWINGS

For more clearly illustrating embodiments of the present application or technical solutions in the conventional technology, the drawing referred to for describing the embodiments or the conventional technology will be briefly described hereinafter. Apparently, the drawings in the following description are only some examples of the present application, and for those skilled in the art, other drawings may be obtained based on the provided drawings without any creative efforts.

The structure, scale, size and the like shown in this specification are only used to match the contents disclosed in this specification for those skilled in the art to understand and read, and are not used to limit the implementation conditions of this application, so it has no technical substantive significance. Any structural modification, proportional relationship change or size adjustment should still fall within the scope of the technical content disclosed in the present application without affecting the effects and objectives that the present application can produce.
Figure 1 is a schematic front view of a laryngeal mask airway provided by an embodiment of the present application.
Figure 2 is a schematic diagram of an airway tube cavity in a laryngeal mask airway provided by an embodiment of the present application.
Figure 3 is a structural schematic diagram of a pharyngeal tube and a video tube cavity of a laryngeal mask airway provided by an embodiment of the present application.
Figure 4 is a left structural schematic diagram of a laryngeal mask airway provided by an embodiment of the present application.
Figure 5 is a right structural schematic diagram of a laryngeal mask airway provided by an embodiment of the present application.

In Figures:

| | | | |
|---|---|---|---|
| 1 | airbag cover | 11 | cover belly |
| 12 | cover back | 13 | cover top drainage port |
| 14 | inflation tube | 2 | extension body |
| 3 | breathing tube cavity | 31 | distal vent |
| 32 | proximal vent | 33 | vent tilted portion |
| 34 | auxiliary vent groove | 4 | esophageal drainage tube cavity |
| 41 | distal esophageal drainage port | 42 | proximal esophageal drainage port |
| 5 | pharyngeal tube | 51 | free section |
| 52 | main body section | 53 | proximal pharyngeal opening |
| 6 | video tube cavity | 61 | blind end transparent window |
| 62 | distal end opening of video tube cavity | | |

### DETAILED DESCRIPTION

The following specific embodiments illustrate the implementation of the present application. Those familiar with the technology can easily understand the other advantages and effects of the present application from the content disclosed in this specification. It is apparent that the described embodiments are only a part of the embodiments according to the present application, rather than all of the embodiments. Based on the embodiments in the present application, all of other embodiments, made by the person skilled in the art without any creative efforts, fall into the scope of the present application.

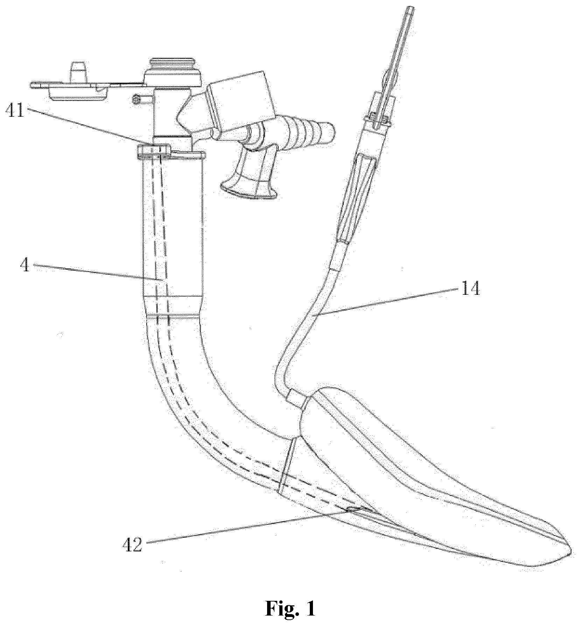

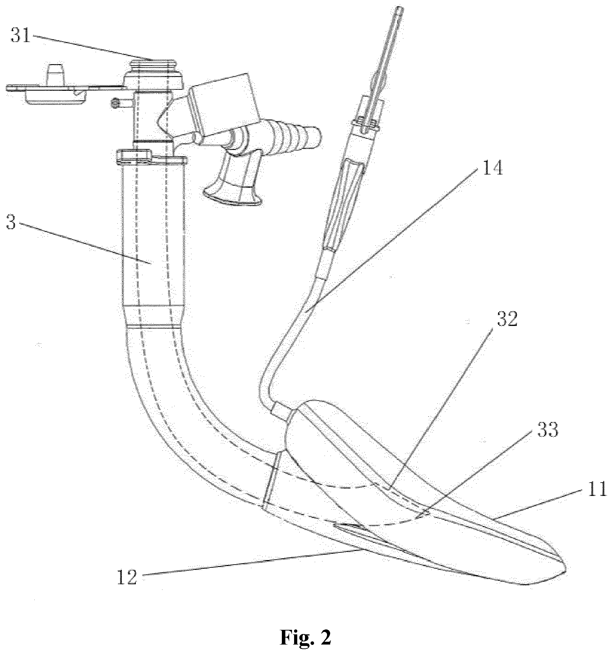

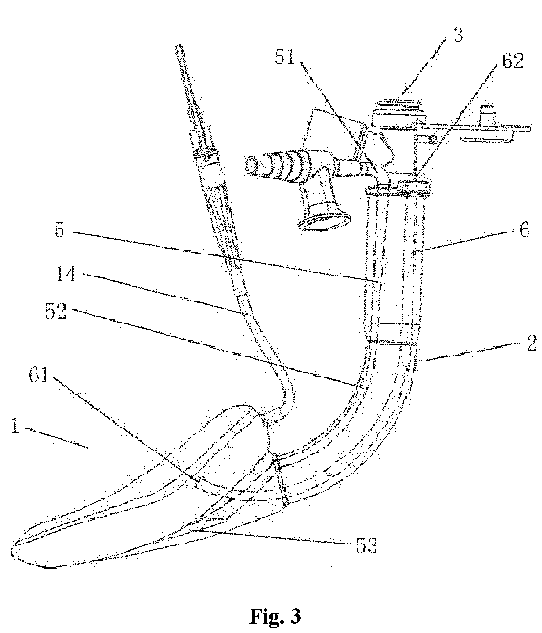

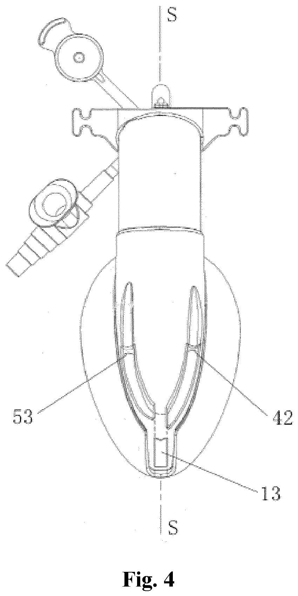

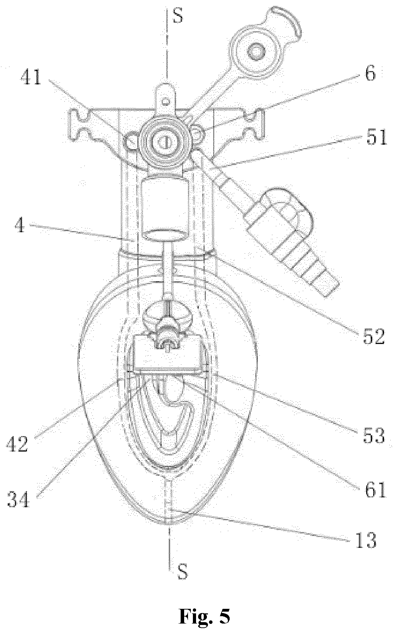

As shown in Figures 1 to 5, it is a laryngeal mask airway provided by an embodiment of the present application. Its main structure is symmetrical left and right and has a central symmetry plane S, including an airbag cover 1 and an extension body 2. The extension body 2 is shaped according to the natural curvature of the throat airway and is arc-shaped, and has a distal end for extending into the oral throat and a proximal end located outside the oral cavity; the extension body 2 has a airway tube cavity 3 extending from the distal end to the proximal end, thereby forming a distal vent 31 and a proximal vent 32 respectively. The airbag cover 1 is arranged at the proximal end of the extension body 2 so as to anastomose with the throat of a patient when in use. The airbag cover 1 has a cover belly 11 forming a concave space and a cover back 12 on the opposite side. The concave space of the airbag cover 1 is in gas communication with the proximal vent 32 of the airway tube cavity 3. The proximal vent 32 is tilted toward the cover belly (that is, the inner side of the arc) with respect to the axis at the proximal end of the airway tube cavity 3, thereby forming a vent tilted portion 33. The airbag cover 1.

The laryngeal mask airway provided by the embodiment of the present application can be directly used for providing respiration for a patient anesthetized during operation. At this time, the laryngeal mask airway is placed into the oral and throat of the patient, and the cover belly of the airbag cover 1 is covered and sealed around the airway glottis. The airway tube cavity 3 can be directly used for providing air to the anesthetized patient during operation, so that the patient can keep normal breathing (for example, through a respirator), and the airway tube cavity is isolated from the esophageal tube, so as to avoid esophageal reflux into the airway.

The laryngeal mask airway provided by the embodiment of the present application can also be used for guiding tracheal intubation through the laryngeal mask. In many cases, the laryngeal mask is not suitable for long-term indwelling use on the patient. At this time, the tracheal intubation can be guided through the laryngeal mask airway tube cavity 3 and inserted into the patient's trachea through the glottis. Under the condition that the laryngeal mask airway is correctly positioned in the patient's mouth and throat, the tracheal intubation can be accurately aligned with the glottis and trachea by using the guiding function of the airway tube cavity 3 of the laryngeal mask airway, thus avoiding the damage to the patient's glottis and throat caused by inaccurate insertion position.

In the use of the laryngeal mask airway provided by the embodiment of the present application, the vent tilted portion 33 is in an upward titled position when the laryngeal mask airway is inserted into the supine oral cavity of a patient.

In the laryngeal mask airway provided by the embodiment of the present application, the purpose of setting the vent tilted portion 33 is to prevent the tissue near the glottis of the throat airway from blocking the proximal vent 32 of the airway tube cavity 3, and to facilitate the correct guidance of the intubation.

During the operation, the patient is in anesthesia, and the throat, especially the tissue near glottis, is paralyzed, which is likely to block the proximal vent 32 of the airway tube cavity 3, thus blocking the patient's breathing. In particular, when the endotracheal intubation is pulled out of the patient's airway through the airway tube cavity 3, it is more likely to cause the tissue near the glottis of the throat airway to block the airway.

Clinical studies have shown that when the angle at which the vent tilted portion 33 is tilted relative to the axis at the distal end of the airway tube cavity 3 is 15 to 45 degrees, which is most beneficial for intubation.

According to the laryngeal mask airway provided by the embodiment of the present application, an auxiliary vent groove 34 can be formed on a part of the wall surface of the proximal vent 32 in the direction opposite to the tilting direction (i.e., toward the cover back). In use, when the laryngeal mask airway is inserted into the supine patient's mouth, the auxiliary vent groove 34 is in a downward concave position.

In the laryngeal mask airway provided by the embodiment of the present application, the purpose of setting the auxiliary vent groove 34 is: when the proximal vent 32 is accidentally blocked by the tissue structure near the glottis of the throat airway, the auxiliary vent groove 34 may not be blocked because it is partially concave from the wall of the proximal vent 32. This further ensures that the proximal vent 32 of the airway tube cavity 3 remains unblocked, thus avoiding airway obstruction. The auxiliary vent groove 34 is located on the left side of the central symmetry plane S. However, its position is not limited to this, and the auxiliary vent groove 34 may also be located on the right side of the central symmetry plane S.

In the laryngeal mask airway provided by the embodiment of the present application, the airway tube cavity 3 can adopt a structure with a variable cross section. The distal vent 31 and the proximal vent 32 of the airway tube cavity 3 are both circular in cross section and have the same diameter. The intermediate portion between the distal vent 31 and the proximal vent 32 has a larger cross sectional size. When endotracheal intubation is performed via laryngeal mask, the diameters of the distal vent 31 and the proximal vent 32 of the airway tube cavity 3 are slightly larger than the diameter of endotracheal intubation.

In the laryngeal mask airway provided by the embodiment of the present application, the distal vent 31 and the proximal vent 32 of the airway tube cavity 3 are both circular in cross section and have the same diameter. The intermediate portion between the distal vent 31 and the proximal vent 32 has an elliptical cross section, and the short axis length of the ellipse is equal to the diameter of the circle. The intermediate portion of the airway tube cavity 3 can also adopt other cross-sectional shapes, as long as the cross-sectional size is larger than the diameter of the distal vent 31 and the proximal vent 32.

The advantages of providing the airway tube cavity 3 with such a cross-sectional change as described above are: during endotracheal intubation, the diameters of the circular cross sections of the distal vent 31 and the proximal vent 32 are equivalent to the diameter of the endotracheal intubation, which can secure and seal the endotracheal intubation well; the transverse dimension of the intermediate portion is larger than the diameter of the intubation, so the resistance during insertion and extraction can be reduced. Such a structure makes the insertion and extraction of the endotracheal intubation more convenient and quick, and reduces the injury and pain of the patient. The laryngeal mask airway provided by the embodiment of the present application further includes an esophageal drainage tube cavity 4. The esophageal drainage tube cavity 4 is used for inserting a gastric tube into the esophagus of a patient up to the stomach of the patient, thereby discharging gas or residual gastric juice in the stomach of the patient. The esophageal drainage tube cavity 4 extends from its distal end to the cover back 12 of the airbag cover 1 in the extension body 2, thereby forming a distal esophageal drainage port 41 and a proximal esophageal drainage port 42. The proximal esophageal drainage port 42 is formed in an open first groove on the upper side of the cover back 12; a cover top drainage port 13 is formed at the top end of the cover back 12, and the first groove and the cover top drainage port 13 are in fluid communication with each other. The laryngeal mask airway provided by the present application further includes a pharyngeal tube 5. The pharyngeal tube 5 is made of flexible material. The pharyngeal tube 5 and the airway tube cavity 3 are not communicated with each other, and are independent of the esophageal drainage tube cavity 4. It includes a main body section 52 integrated in the extension body 2 and a free section 51 extending from the proximal end of the extension body 2 to the outside of the extension body 2. The main body section 52 extends from the distal end to the proximal end in the extension body 2, and forms a proximal pharyngeal opening 53 at the proximal end. In the extension body 2, the main body section 52 of the pharyngeal tube 5 is located on the side opposite to the esophageal drainage tube cavity 4 in the left-right direction, and the proximal pharyngeal opening 53 is shaped into an open second groove located on the inner side of the cover edge on the back side of the airbag cover 1; the first groove, the second groove and the cover top drainage port are in fluid communication with each other to form a three-way structure.

In the laryngeal mask airway provided by the embodiment of the present application, the top end of the airbag cover 1 forms a sealing joint just around the esophageal orifice when the throat of the patient is mounted in place, at the same time, the cover top drainage port 13 is aligned with the esophageal orifice.

In the laryngeal mask airway provided by the embodiment of the present application, in the extension body 2, the esophageal drainage tube cavity 4 may be located on the left or right side of the airway tube cavity 3. As an example, in the structure shown in Figure 1, the esophageal drainage tube cavity 4 may be located on the left side of the airway tube cavity 3.

The purpose of setting the pharyngeal tube 5 is to suck out the oral secretion, gastric reflux and bleeding blood around the airbag cover 1, keep the throat around the airbag cover 1 clean, and avoid the secretion, gastric reflux and bleeding blood accidentally entering the airway and affecting the operation. To achieve the object, the free section 51 of the pharyngeal tube 5 may be connected with a suction device.

With the above three-way structure, the gastric reflux, oral secretion and bleeding blood around the airbag cover 1 may be pumped out regardless of whether the stomach tube is inserted, which is more conductive to keeping the throat around the airbag cover 1 clean.

The laryngeal mask airway provided by the present application may further include a video tube cavity 6, which is used for inserting video elements (such as video cameras, lighting elements, etc.) and their leads, so as to monitor the patient's internal condition in front of the laryngeal mask airway at any time. The video tube cavity 6 extends from its distal end to its proximal end in the extension body 2, and is not communicated with other tube cavities, thereby forming a distal end opening of video tube cavity 62 at the distal end and a blind end transparent window 61 for accommodating and protecting video elements at the proximal end.

The blind end transparent window 61 has good light transmittance to ensure the light input of the camera. The blind end transparent window 61 also has good flexibility and extensibility, so as to fit the video elements evenly, thus avoiding distortion. If the blind end transparent window 61 cannot fit well with the video element, the gap between the blind end transparent window 61 and the video element may cause uneven refraction of the pipeline, which may adversely affect the imaging quality.

In the laryngeal mask airway provided by the embodiment of the present application, the blind end transparent window 61 may be a separately manufactured part and mounted at the distal end of the video tube cavity 6. The use of a separately manufactured blind end transparent window 61 is beneficial to ensure the above good light transmittance, flexibility and extensibility of the blind end transparent window 61, while these performance requirements are not required for the other parts of the video tube cavity 6. Therefore, it is advantageous to manufacture the high-quality video tube cavity 6 at a lower cost, and takes into account the performance requirements of the whole extension body 2.

The blind end transparent window 61 may be located on the left or right side of the proximal vent 32. Since the patient's mouth and airway are not straight in the up and down direction (when the patient is lying on his back), but in a certain arc, choosing the blind end transparent window 61 on the side of the proximal vent 32 helps to extend the camera's viewing angle range. This is very beneficial to the correct placement of the laryngeal mask airway and the accuracy of intubation. The blind end transparent window 61 is located on the right side of the proximal vent 32, but it is also possible to locate on the left side.

In the laryngeal mask airway provided by the embodiment of the present application, the blind end transparent window 61 is located on the other side of the auxiliary vent groove 34 relative to the central symmetry plane S. This structure helps to avoid the interference between the blind end transparent window 61 and the auxiliary vent groove 34 in a narrow space, and makes the design and manufacture easier.

In the laryngeal mask airway provided by the embodiment of the present application, in the extension body 2, the video tube cavity 6 is located on the other side of the esophageal drainage tube cavity 4 relative to the central symmetry plane S. This design makes the multi-functional laryngeal mask more symmetrical and balanced in structure, avoids mutual interference between the operation of the video tube cavity 6 and the esophageal drainage tube cavity 4 in use, and makes the design and manufacture easier to realize. In the laryngeal mask airway provided by the present application, the airbag cover 1 can be an inflatable airbag cover, and the laryngeal mask airway further includes an inflation tube 14 connected to the airbag cover. The inflation tube may be connected with an inflation device, such as an air pump or an inflation cylinder (not shown in the Figure). The airbag cover is made of flexible material, and materials with Shore A hardness lower than 40, preferably lower than 30, and particularly preferably lower than 20 may be used.

With the inflatable airbag cover made of flexible material, the airbag cover 1 may better fit the throat of the patient, thus better sealing the airway and esophagus of the patient, avoiding mutual leakage between them, preventing foreign bodies from entering the trachea, and preventing breathing gas from entering the stomach.

The laryngeal mask airway provided by the present application has many advantages: as all the tube cavities are integrated in the extension body, the whole laryngeal mask has good integrity and streamline, which is helpful to conveniently and quickly place or take out the laryngeal mask at the throat of a patient, which reduces operation errors and helps to clean and tidy the laryngeal mask. Since the tube cavities are independent of each other, the operations for each tube cavity can be operated without interference.

Through the above integrated structure, all functions such as breathing and endotracheal intubation, esophageal drainage, oral and throat secretion drainage, video capture and so on can be integrated in the same laryngeal mask, or only part of the functions can be integrated.

Because the vent tilted portion is formed at the proximal vent, it is helpful to guide the endotracheal intubation to align with the glottis airway of the patient, thus helping to insert the endotracheal intubation accurately. In addition, the vent tilted portion also helps to prevent the proximal vent from being blocked by the tissue near the glottis of the patient, thus keeping the patient breathing smoothly.

Because the concave auxiliary vent groove is formed on the wall surface of the proximal vent in the direction opposite to the lifting direction of the vent tilted portion, even if the proximal vent is accidentally blocked, the patient's breathing can still be kept smooth.

Because the distal vent and the proximal vent of the airway tube cavity are round in cross section and have the same diameter, and the intermediate portion between the distal vent and the proximal vent has a larger cross section size, the resistance of the endotracheal intubation when inserted into the airway tube cavity can be reduced without affecting the positioning of the endotracheal intubation in the airway tube cavity.

In the laryngeal mask airway provided by the embodiment of the present application, through the three-way structure formed by the first groove, the second groove and the drainage port at the top of the mask, the secretion or blood near the mouth and throat and the gastric juice flowing back from the stomach can be sucked and discharged to the outside of the patient through the pharyngeal tube.

In the laryngeal mask airway provided by the embodiment of the present application, by setting the blind end transparent window of the video tube cavity on the left or right side of the proximal vent, the shooting angle and range of the video element can be effectively extended, thus providing a good field of view for doctors.

The laryngeal mask airway provided by the embodiment of the present application has the following advantages:
(1) The laryngeal mask airway provided by the embodiment of the present application can quickly, safely and effectively establish the artificial airway, which has less damage and simple operation, and can improve the management level of medical staff on the artificial airway.
(2) The laryngeal mask airway provided by the embodiment of the present application has esophageal drainage function, independent esophageal drainage cavity structure design, prevents aspiration caused by esophageal flux, and is safer in clinical application;
(3) The laryngeal mask airway provided by the embodiment of the present application has the function of throat drainage, and backpack drainage is mainly used in otolaryngology surgery, which can carry out real-time drainage of oropharynx and nasopharynx, reduce the occurrence of aspiration and improve the safety requirements of clinical application;
(4) The laryngeal mask airway provided by the embodiment of the present application has the function of intubation through the laryngeal mask, and the laryngeal mask tube refers to the structural characteristics of the human oral cavity and the laryngopharynx. The intubation replacement function effectively establishes the intubation channel, and the guiding device is designed to better assist the intubation and improve the success rate of intubation. Especially in the difficult airway, the clinical application value is higher.
(5) The laryngeal mask airway provided by the embodiment of the present application has the function of visual monitoring, and prevents cross-infection caused by repeated use of visual devices, monitor the airway in real time, and improve airway management.

In the laryngeal mask airway provided by the embodiment of the present application, the laryngeal bag is made into a bag which conforms to the anatomical structure in advance, where the airway is simple, effective, safe and convenient in clinical use, and has high cost-effectiveness in production and manufacture. In addition, when the traditional artificial airway can't improve the quality of airway management and hinder the operation, in order to effectively meet the requirements of various clinical situations, such as ophthalmology, otolaryngology, ENT, or head surgery, neck surgery, maxillary surgery, this type of laryngeal mask is preferred. The reason is that it can flexibly and effectively open the surgical field to continue to provide breathing for the patient without interfering with the operation, and can effectively provide multiple airway safety management programs. In summary, laryngeal mask airways have many advantages in certain applications, but we have found that in many applications, the use of inter-buccal fixators can also be a disadvantage. For example, in the use of traditional laryngeal mask, when inserting endotracheal intubation or esophageal drainage tube through laryngeal mask, doctors can't see the human tissue in front of the insertion path, so it is easy to cause injury to patients. However, with the improvement of medical clinical requirements, the demand for throat real-time monitoring and intubation replacement function is increasingly strong, and the traditional laryngeal mask has no backpack drainage function in otolaryngology clinical surgery, which greatly affects the clinical application effect of laryngeal mask and limits the cross application among departments. The development of visual and multipurpose drainage laryngeal mask greatly improves the effectiveness and safety of clinical application, and is easy to popularize and apply in clinical anesthesia.

Although the present application has been described in detail above with general descriptions and specific embodiments, some modifications or improvements can be made on the basis of the present application, which is apparent to those skilled in the art.

## Claims

1. A laryngeal mask airway, comprising an airbag cover (1) and an extension body (2), wherein the extension body (2) is shaped according to a natural curvature of an oral cavity and a throat airway and is of an arc shape, and has a proximal end for extending into a laryngopharynx of the oral cavity and a distal end located outside the oral cavity; and
the extension body (2) has an airway tube cavity (3) extending from the distal end to the proximal end, thereby forming a distal vent (31) and a proximal vent (32) respectively, and the airbag cover (1) is arranged at the proximal end and has a cover belly (11) forming a concave space and a cover back (12) on the opposite side; the cover belly (11), having the concave space, of the airbag cover (1) is communicated with the proximal vent (32) of the airway tube cavity (3); wherein the proximal vent (32) is tilted toward the cover belly (11) with respect to an axis at the proximal end of the airway tube cavity (3), thereby forming a vent tilted portion (33); and
the laryngeal mask airway further comprises a video tube cavity (6), and the video tube cavity (6) extends from the distal end to the proximal end in the extension body (2), thereby forming a distal end opening of video tube cavity (62) at the distal end and a blind end transparent window (61) at the proximal end, and the blind end transparent window (61) is located at one side of the proximal vent (32),
**characterized by** further comprising an esophageal drainage tube cavity (4), and the esophageal drainage tube cavity (4) extends from the distal end to the cover back (12) of the airbag cover (1) in the extension body (2), thereby forming a proximal esophageal drainage port (42) and a distal esophageal drainage port (41); and
a proximal part of the esophageal drainage tube cavity (4) is embedded in an inner side of the esophageal drainage port, and the proximal end of the esophageal drainage tube cavity (4) is matched with a flexible material to perform a sealing anastomosis with the esophageal opening of the laryngopharynx; and
the distal end is opened outside the lip, and the proximal esophageal drainage port (42) of the esophageal drainage tube cavity (4) is formed in an open first groove on the upper side of the cover back (12); a cover top drainage port (13) is formed at the top end of the cover back (12) of the airbag cover (1), and the first groove and the cover top drainage port (13) are in fluid communication with each other,
further comprising a pharyngeal tube (5), the pharyngeal tube (5) is made of flexible material; and
the pharyngeal tube (5) and the airway tube cavity (3) are not communicated with each other, and are independent of the esophageal drainage tube cavity (4); the pharyngeal tube (5) comprises a main body section (52) integrated in the extension body (2) and a free section (51) extending from the distal end of the extension body (2) to an outside of the extension body (2); a tail end of the free section (51) is matched with a standard drainage interface, and the main body section (52) extends from the distal end to the proximal end in the extension body (2), and forms a proximal pharyngeal opening (53) at the proximal end,
wherein, in the extension body (2) , the main body section (52) is located on the side opposite to the esophageal drainage tube cavity (4) in a left-right direction, and the proximal pharyngeal opening (53) is shaped into an open second groove located on an inner side of the cover edge on the back side of the airbag cover (1), the first groove, the second groove and the cover top drainage port are in fluid communication with each other to form a three-way structure.

2. The laryngeal mask airway according to claim 1, wherein the angle included between the vent tilted portion (33) and the axis at the proximal end of the airway tube cavity (3) is between 15 and 45 degrees.

3. The laryngeal mask airway according to claim 1, wherein a distal end of the vent tilted portion (33) adopts a streamlined arc design and an endotracheal intubation guide groove structure is formed at a proximal end of the vent tilted portion (33),
which is configured to assist in guiding the endotracheal intubation into the patient's airway through the airway tube cavity (3).

4. The laryngeal mask airway according to claim 1, wherein a part of a side wall surface of the proximal vent (32) is concave in a direction toward the cover back (12), thereby forming an auxiliary vent groove (34).

5. The laryngeal mask airway according to claim 1, wherein the proximal vent (32) and the distal vent (31) of the airway tube cavity (3) are both circular in cross section and have a same diameter, a cross-sectional dimension of an intermediate portion between the distal vent (31) and the proximal vent (32) is larger than a cross-sectional dimension of the proximal vent (32) of the airway tube cavity (3).

6. The laryngeal mask airway according to claim 5, wherein an intermediate portion between the proximal vent (32) and the distal vent (31) has an elliptical shape in cross section and a short axis length of the elliptical shape is equal to a diameter of the circle.

7. The laryngeal mask airway according to claim 1, wherein the airbag cover (1) is an inflatable airbag cover, the laryngeal mask airway further comprises an inflation tube connected to the airbag cover (1), the airbag cover (1) is made of flexible material, and the hardness of which is lower than the Shore A hardness of 30.

## Patentansprüche

1. Larynxmasken-Luftweg, umfassend eine Luftkissenabdeckung (1) und einen Verlängerungskörper (2), wobei der Verlängerungskörper (2) gemäß einer natürlichen Krümmung einer Mundhöhle und eines Rachenraums geformt und bogenförmig ist, und ein proximales Ende, welches sich in einen Laryngopharynx der Mundhöhle erstreckt, und ein distales Ende, welches sich außerhalb der Mundhöhle befindet, aufweist; und
wobei der Verlängerungskörper (2) einen Luftwegtubushohlraum (3) aufweist, welcher sich von dem distalen Ende zu dem proximalen Ende erstreckt, wodurch eine distale Entlüftung (31) bzw. eine proximale Entlüftung (32) gebildet werden, und wobei die Luftkissenabdeckung (1) an dem proximalen Ende angeordnet ist und einen einen konkaven Raum bildenden Abdeckbauch (11) und auf der gegenüberliegenden Seite einen Abdeckrücken (12) aufweist; wobei der den konkaven Raum aufweisende Abdeckbauch (11) der Luftkissenabdeckung (1) mit der proximalen Entlüftung (32) des Luftwegtubushohlraums (3) in Verbindung steht; wobei die proximale Entlüftung (32) in Bezug auf eine Achse an dem proximalen Ende des Luftwegtubushohlraums (3) zu dem Abdeckbauch (11) hin geneigt ist, wodurch ein geneigter Entlüftungsabschnitt (33) gebildet wird; und
wobei der Larynxmasken-Luftweg weiter einen Videotubushohlraum (6) umfasst und sich der Videotubushohlraum (6) von dem distalen Ende zu dem proximalen Ende in dem Verlängerungskörper (2) erstreckt, wodurch eine distale Endöffnung des Videotubushohlraums (62) an dem distalen Ende und ein transparentes Fenster (61) mit blindem Ende an dem proximalen Ende gebildet werden, und wobei das transparente Fenster (61) mit blindem Ende auf einer Seite der proximalen Entlüftung (32) angeordnet ist,
**dadurch gekennzeichnet, dass** er weiter einen Ösophagus-Drainagetubushohlraum (4) umfasst, wobei sich der Ösophagus-Drainagetubushohlraum (4) von dem distalen Ende bis zu dem Abdeckrücken (12) der Luftkissenabdeckung (1) in dem Verlängerungskörper (2) erstreckt, wodurch eine proximale Ösophagus-Drainageöffnung (42) und eine distale Ösophagus-Drainageöffnung (41) gebildet werden; und
ein proximaler Teil des Ösophagus-Drainagetubushohlraums (4) in eine Innenseite der Ösophagus-Drainageöffnung eingebettet ist und das proximale Ende des Ösophagus-Drainagetubushohlraums (4) auf ein flexibles Material abgestimmt ist, um eine abdichtende Anastomose mit der Ösophagusöffnung des Laryngopharynx durchzuführen; und
das distale Ende außerhalb der Lippe geöffnet ist und die proximale Ösophagus-Drainageöffnung (42) des Ösophagus-Drainagetubushohlraums (4) in einer offenen ersten Nut auf der Oberseite des Abdeckrückens (12) ausgebildet ist; an dem oberen Ende der Drainageöffnung (12) der Luftkissenabdeckung (1) eine obere Abdeckdrainageöffnung (13) ausgebildet ist und die erste Nut und die obere Abdeckdrainageöffnung (13) in Fluidverbindung miteinander stehen,
weiter umfassend einen Rachentubus (5), wobei der Rachentubus (5) aus flexiblem Material hergestellt ist; und
der Rachentubus (5) und der Luftwegtubushohlraum (3) nicht miteinander in Verbindung stehen und unabhängig von dem Ösophagus-Drainagetubushohlraum (4) sind; der Rachentubus (5) einen in den Verlängerungskörper (2) integrierten Hauptkörperabschnitt (52) und einen freien Abschnitt (51) umfasst, welcher sich von dem distalen Ende des Verlängerungskörpers (2) zu einer Außenseite des Verlängerungskörpers (2) erstreckt; ein hinteres Ende des freien Abschnitts (51) auf eine Standard-Drainageschnittstelle abgestimmt ist und sich der Hauptkörperabschnitt (52) von dem distalen Ende zu dem proximalen Ende in dem Verlängerungskörper (2) erstreckt und eine proximale Rachenöffnung (53) an dem proximalen Ende bildet,
wobei sich in dem Verlängerungskörper (2) der Hauptkörperabschnitt (52) auf der Seite befindet, welche dem Ösophagus-Drainagetubushohlraum (4) in einer Links-Rechts-Richtung gegenüberliegt, und die proximale Rachenöffnung (53) in eine offene zweite Nut geformt ist, welche an einer Innenseite des Abdeckungsrandes auf der Rückseite der Luftkissenabdeckung (1) angeordnet ist, wobei die erste Nut, die zweite Nut und die obere Abdeckdrainageöffnung in Fluidverbindung miteinander stehen, um eine Dreiwegstruktur zu bilden.

2. Larynxmasken-Luftweg nach Anspruch 1, wobei der Winkel zwischen dem geneigten Entlüftungsabschnitt (33) und der Achse an dem proximalen Ende des Luftwegtubushohlraums (3) zwischen 15 und 45 Grad beträgt.

3. Larynxmasken-Luftweg nach Anspruch 1, wobei ein distales Ende des geneigten Entlüftungsabschnitts (33) ein stromlinienförmiges Bogendesign annimmt und an einem proximalen Ende des geneigten Entlüftungsabschnitts (33) eine Nutenführungsstruktur für die endotracheale Intubation ausgebildet ist,
welche dazu konfiguriert ist, die Führung der endotrachealen Intubation in die Luftwege des Patienten durch den Luftwegtubushohlraum (3) zu unterstützen.

4. Larynxmasken-Luftweg nach Anspruch 1, wobei ein Teil einer Seitenwandoberfläche der proximalen Entlüftung (32) in Richtung des Abdeckrückens (12) konkav ist und dadurch eine Hilfsentlüftungsnut (34) bildet.

5. Larynxmasken-Luftweg nach Anspruch 1, wobei die proximale Entlüftung (32) und die distale Entlüftung (31) des Luftwegtubushohlraums (3) beide kreisförmig im Querschnitt sind und den gleichen Durchmesser aufweisen, wobei eine Querschnittsabmessung eines Zwischenabschnitts zwischen der distalen Entlüftung (31) und der proximalen Entlüftung (32) größer als eine Querschnittsabmessung der proximalen Entlüftung (32) des Luftwegtubushohlraums (3) ist.

6. Larynxmasken-Luftweg nach Anspruch 5, wobei ein Zwischenabschnitt zwischen der proximalen Entlüftung (32) und der distalen Entlüftung (31) im Querschnitt eine elliptische Form aufweist und eine kurze Achsenlänge der elliptischen Form gleich einem Durchmesser des Kreises ist.

7. Larynxmasken-Luftweg nach Anspruch 1, wobei die Luftkissenabdeckung (1) eine aufblasbare Luftkissenabdeckung ist, wobei der Larynxmasken-Luftweg weiter einen Aufblastubus umfasst, welcher mit der Luftkissenabdeckung (1) verbunden ist, wobei die Luftkissenabdeckung (1) aus flexiblem Material hergestellt ist und die Härte desselben unter der Shore-A-Härte von 30 liegt.

## Revendications

1. Masque laryngé, comprenant un couvercle d'airbag (1) et un corps d'extension (2), dans lequel le corps d'extension (2) est façonné selon une courbure naturelle d'une cavité buccale et d'une voie respiratoire de la gorge et est en forme d'arc, et présente une extrémité proximale pour s'étendre dans un laryngopharynx de la cavité buccale et une extrémité distale située à l'extérieur de la cavité buccale ; et
le corps d'extension (2) présente une cavité de tube respiratoire (3) s'étendant depuis l'extrémité distale jusqu'à l'extrémité proximale, formant ainsi respectivement un évent distal (31) et un évent proximal (32), et le couvercle d'airbag (1) est agencé au niveau de l'extrémité proximale et présente un ventre de couvercle (11) formant un espace concave et un dos de couvercle (12) sur le côté opposé ; le ventre de couvercle (11), présentant l'espace concave, du couvercle d'airbag (1) est en communication avec l'évent proximal (32) de la cavité de tube respiratoire (3) ; dans lequel l'évent proximal (32) est incliné vers le ventre de couvercle (11) par rapport à un axe au niveau de l'extrémité proximale de la cavité de tube respiratoire (3), formant ainsi une partie inclinée d'évent (33) ;
le masque laryngé comprend en outre une cavité de tube vidéo (6) et la cavité de tube vidéo (6) s'étend depuis l'extrémité distale jusqu'à l'extrémité proximale dans le corps d'extension (2), formant ainsi une ouverture d'extrémité distale de cavité de tube vidéo ( 62) au niveau de l'extrémité distale et une fenêtre transparente d'extrémité borgne (61) au niveau de l'extrémité proximale, et la fenêtre transparente d'extrémité borgne (61) est située sur un côté de l'évent proximal (32),
**caractérisé en ce qu'**il comprend en outre une cavité de tube de drainage oesophagien (4) et la cavité de tube de drainage oesophagien (4) s'étend depuis l'extrémité distale jusqu'au dos de couvercle (12) du couvercle d'airbag (1) dans le corps d'extension (2), formant ainsi un orifice de drainage oesophagien proximal (42) et un orifice de drainage oesophagien distal (41) ; et
une partie proximale de la cavité de tube de drainage oesophagien (4) est intégrée dans un côté interne de l'orifice de drainage oesophagien et l'extrémité proximale de la cavité de tube de drainage oesophagien (4) est mise en correspondance avec un matériau flexible pour réaliser une anastomose étanche avec l'ouverture oesophagienne du laryngopharynx ; et
l'extrémité distale est ouverte à l'extérieur de la lèvre et l'orifice de drainage oesophagien proximal (42) de la cavité de tube de drainage oesophagien (4) est formé dans une première rainure ouverte sur le côté supérieur du dos de couvercle (12) ; un orifice de drainage de sommet de couvercle (13) est formé au niveau de l'extrémité supérieure du dos de couvercle (12) du couvercle d'airbag (1) et la première rainure et l'orifice de drainage de sommet de couvercle (13) sont en communication fluidique l'un avec l'autre,
comprenant en outre un tube pharyngé (5), le tube pharyngé (5) est composé d'un matériau flexible ; et
le tube pharyngé (5) et la cavité de tube respiratoire (3) ne communiquent pas entre eux et sont indépendants de la cavité de tube de drainage oesophagien (4) ; le tube pharyngé (5) comprend une section de corps principal (52) intégrée dans le corps d'extension (2) et une section libre (51) s'étendant depuis l'extrémité distale du corps d'extension (2) vers un extérieur du corps d'extension (2) ; une extrémité arrière de la section libre (51) est mise en correspondance avec une interface de drainage standard, et la section de corps principal (52) s'étend depuis l'extrémité distale jusqu'à l'extrémité proximale dans le corps d'extension (2) et forme une ouverture pharyngée proximale (53) au niveau de l'extrémité proximale,
dans lequel, dans le corps d'extension (2), la section de corps principal (52) est située sur le côté opposé à la cavité de tube de drainage oesophagien (4) dans une direction gauche-droite et l'ouverture pharyngée proximale (53) est façonnée en une seconde rainure ouverte située sur un côté interne du bord du couvercle sur le côté arrière du couvercle d'airbag (1), la première rainure, la seconde rainure et l'orifice de drainage de sommet de couvercle sont en communication fluidique les uns avec les autres pour former une structure à trois voies.

2. Masque laryngé selon la revendication 1, dans lequel l'angle inclus entre la partie inclinée d'évent (33) et l'axe au niveau de l'extrémité proximale de la cavité de tube respiratoire (3) est compris entre 15 et 45 degrés.

3. Masque laryngé selon la revendication 1, dans lequel une extrémité distale de la partie inclinée d'évent (33) adopte une conception en arc profilé et une structure de rainure de guidage d'intubation endotrachéale est formée au niveau d'une extrémité proximale de la partie inclinée d'évent (33),
qui est configurée pour aider à guider l'intubation endotrachéale dans les voies respiratoires du patient par la cavité de tube respiratoire (3).

4. Masque laryngé selon la revendication 1, dans lequel une partie d'une surface de paroi latérale de l'évent proximal (32) est concave dans une direction vers le dos de couvercle (12), formant ainsi une rainure d'évent auxiliaire (34).

5. Masque laryngé selon la revendication 1, dans lequel l'évent proximal (32) et l'évent distal (31) de la cavité de tube respiratoire (3) sont tous deux circulaires en coupe transversale et présentent un même diamètre, une dimension en coupe transversale d'une partie intermédiaire entre l'évent distal (31) et l'évent proximal (32) est plus grande qu'une dimension en coupe transversale de l'évent proximal (32) de la cavité de tube respiratoire (3).

6. Masque laryngé selon la revendication 5, dans lequel une partie intermédiaire entre l'évent proximal (32) et l'évent distal (31) présente une forme elliptique en coupe transversale et une longueur d'axe court de la forme elliptique est égale à un diamètre du cercle.

7. Masque laryngé selon la revendication 1, dans lequel le couvercle d'airbag (1) est un couvercle d'airbag gonflable, le masque laryngé comprend en outre un tube de gonflage relié au couvercle d'airbag (1), le couvercle d'airbag (1) est composé d'un matériau flexible dont la dureté est inférieure à la dureté Shore A de 30.
